# EUROPEAN PATENT SPECIFICATION

(11) **EP 1 508 026 B1**
(45) Date of publication and mention of the grant of the patent: **01.07.2009**
(21) Application number: 03730390.6
(22) Date of filing: 27.05.2003
(51) Int. Cl.: G01N 1/30

(54) **COMPOSITION FOR THE PREPARATION OF HISTOLOGICAL, AUTOPSICAL, CYTOLOGICAL SAMPLES**
ZUSAMMENSETZUNG FÜR DIE HERSTELLUNG VON HISTOLOGISCHEN, AUTOPSISCHEN UND ZYTOLOGISCHEN PROBEN
COMPOSITION POUR LA PREPARATION D'ECHANTILLONS HISTOLOGIQUES, CYTOLOGIQUES ET D'AUTOPSIE

(30) Priority: 28.05.2002 EP 02425337; 23.12.2002 EP 02028892
(43) Date of publication of application: 23.02.2005
(60) Divisional application: 09001427.5
(73) Proprietor: Diapath S.p.A., 24057 Martinengo (BG) (IT)
(72) Inventor: MOSCONI, Gianfilippo, I-60039 Staffolo (IT); BIANCHI, Monica, I-24060 Casazza (IT)
(74) Representative: Trupiano, Federica
(86) International application number: PCT/IB2003/002029
(87) International publication number: WO 2003/100384

(56) References cited:
- EP-A- 0 822 403
- EP-A- 1 195 594
- WYNNCHUK MARIA: "Evaluation of xylene substitutes for paraffin tissue processing." JOURNAL OF HISTOTECHNOLOGY, vol. 17, no. 2, 1994, pages 143-149, XP008009402 ISSN: 0147-8885
- WHALEN J D ET AL: "Xylene substitutes in frozen sections." DERMATOLOGIC SURGERY: OFFICIAL PUBLICATION FOR AMERICAN SOCIETY FOR DERMATOLOGIC SURGERY [ET AL.]. UNITED STATES MAR 1995, vol. 21, no. 3, March 1995 (1995-03), pages 241-242, XP008009400 ISSN: 1076-0512

## Description

### SUBJECT OF THE INVENTION

The present invention concerns a composition and its use for the preparation of histological, autoptic, cytological and similar samples for examination.

### STATE OF THE ART

As Is known, the analysis of histological, autoptic, cytological and similar samples is of fundamental importance in modern medicine, not only because it ensures accurate and early diagnosis of numerous pathologies, but also because it permits complex studies to be performed that contribute to progress in the sector. Naturally the samples to be analysed must be treated and prepared according to precise protocols, in order to guarantee optimal preservation, analysis reproducibility and reliability of the results.

At present, in the case of a sample taken from a part that has been operated on or from a post-mortem examination, previously fixed, (for example a tissue sample) the first stage in preparation of the sample for examination consists of a so-called processing phase designed to prepare the sample for inclusion In a medium of appropriate consistency to confer the necessary rigidity for cutting by means of blades. The inclusion material which today best satisfies these characteristics is paraffin, but it cannot be mixed with the majority of fixatives used. For this reason, in order for the tissue to be included in paraffin and then cut according to requirements, it must undergo various treatments, all pertaining to the processing phase, that can be summarised as follows:
- Dehydration phase, in which the water contained in the sample is replaced by anhydrous reagents.
- Clarification phase, in which the anhydrous reagent is replaced by a component (clarifying medium) that can be mixed both with the anhydrous reagent and with the inclusion medium.
- Impregnation phase, in which the clarifying medium is replaced by the impregnation medium (or inclusion medium).

The first phase provides for removal of the watery reagents used in the initial phase for fixing the sample as soon as it has been taken. Numerous dehydrating agents can be used, but preferably ethyl alcohol is used in increasing concentrations, i.e. the sample is first treated with 70% ethyl alcohol, then with 95% ethyl alcohol and then again at 100%. Since the most commonly used dehydrating agents are not soluble in paraffin (the medium in which the sample must be included), they must be replaced by a component that is soluble both in the dehydrating agent and in the impregnating agent. In this phase xylene (or xylol, dimethylbenzene) is commonly used which is an aromatic hydrocarbon consisting of a mixture of ortho, meta and para isomers, in a flammable liquid form, very volatile and potentially highly toxic. The potential toxicity of xylene is a constant problem for users. The aspecific effect on the central nervous system is manifested initially by nausea and gastro-intestinal problems and, if the intoxication persists, dizziness, stupor and vomiting. Inhalation causes irritation of the respiratory mucous membranes and probable pulmonary edema. In the case of chronic exposure, alterations to the central nervous system, the leucocytic formula, cardiac functions and, above all, a potential carcinogenicity have been observed. Again according to the known art, the processing phase is generally followed by a phase of inclusion in paraffin, a cutting phase and a staining phase. The latter provides for elimination of the paraffin (which has been used only as a support for the cutting) by treatment with xylene, a rehydration phase with achohols in varying titres (100%, 95%, 70%), staining of the sample, another dehydration stage (70%, 95%, 100%) and, lastly, further treatment with xylene to eliminate the dehydrating agent.

Also in the case of analysis of cytological samples, where inclusion in paraffin is not scheduled or necessary, the repeated use of xylene is required since, also in these cases, the sample undergoes numerous hydration and dehydration phases. It is therefore evident that the repeated use of xylene within a sequence for the preparation of histological and/or autoptic samples for examination involves a certain risk for the operator, also in cases in which most of the sequences are performed by an automated system.

Numerous studies have been carried out to find alternatives to xylene.

EP 1195594 describes a mounting method for microscope analysis samples that comprises a solution of at least one methacrylate resin in an organic solvent. The organic solvent can be advantageously chosen from the saturated hydrocarbons, if necessary mixed with one or more alcohols. The treatment medium is used in particular for application of covers on slides for microscope analysis and to improve the preservation of material stored in archives. EP 0822403 describes a procedure for the processing of organic tissues for analysis, which provides for dehydration and clarification of the sample performed simultaneously using a mixture consisting of a dehydrating agent and a clarification agent. According to the description, the dehydrating agent can be a mixture of ethanol and isopropanol, for example, while the clarifying agent can be an aliphatic hydrocarbon, such as octane. The simultaneous dehydration and clarification phases of the sample are performed under pressure, for example 300-500 mbar, and at a temperature between 70°C and 90°C. EP 0822403 also describes the use of a microwave device for performing the phases indicated above in the appropriate pressure and temperature conditions. It also describes equipment for performing said procedure which comprises a microwave heating device.

According to the invention referred to, heating of the sample to be analysed to the specified temperatures during the simultaneous dehydration and clarification phases is sufficient to generate a pressure that provides a good result at the end of the treatment cycle. It is evident, however, that heating of the sample must not be performed at temperatures higher than those indicated, otherwise the sample will dry out and its cells will be destroyed; likewise, for the same reason, it must not be performed at pressures that are too high. On the other hand, temperatures and pressure below the values indicated do not permit efficient processing of the samples and give unsatisfactory results, with samples that are difficult to analyse and unreliable results. In addition to the use of specific microwave equipment, essential for success of the processing phase, the procedure as described in EP 0822403 therefore requires the operator to pay particular attention to the pressure and temperature conditions at which the operations and sample treatment are performed.

### OBJECT OF THE INVENTION

The aim of the present invention is to make available a composition for the preparation of histological, autoptic, cytological and similar samples that is not carcinogenic, that has a low toxicity level and that is quick and easy to implement. Another aim of this invention is to make available a composition for the preparation of histological, autoptic, cytological and similar samples which eliminates numerous stages that were obligatory up to now in preparation of the sample for examination, which does not involve the use of any additional equipment with respect to the traditional method and which, in some cases, also permits saving in preparation time. A further aim of the present invention is to make available a composition for the preparation of histological, autoptic, cytological and similar samples that replaces the various reagents with a reduced risk of confusion and therefore error on the part of the operator, and that does not involve any heating phase or treatment of the sample with additional equipment in the dehydration and clarification phases. Another aim of the present invention is to make available a method for the preparation of histological, autoptic and cytological samples for examination that is quick, efficient and reliable, that does not involve risks for the operators and that ensures optimal preservation of the sample with consequent reproducibility and reliability of the results deriving from examination of the sample.

### DESCRIPTION

These and other aims and related advantages which will be better illustrated by the following description are achieved by a composition for the preparation of histological, autoptic, cytological and similar samples for examination which comprises octane 60% by volume, isopropyl alcohol 10% by volume and ethyl alcohol 30% by volume. It is intended that the overall percentage of the components is referred to 100 considering the whole composition. In this case, the composition allows for dehydration and clarification of the sample to be analysed without requiring the use of any additional equipment and without having to apply to the sample treated as above particular temperature and/or pressure conditions. In fact, at the above concentrations, the composition according to the invention can be used at ambient temperature and pressure and provides perfectly treatable samples with consequent high reliability of results and considerable convenience and simplicity of use. Since no additional equipment is scheduled, the phases are easy to implement and even a non-expert operator can easily and successfully perform processing of the sample for analysis.

A procedure for preparation of the composition according to the invention provides for mixing of the components until they are fully blended.

The above composition according to the present invention is advantageously used in the preparation of histological, autoptic, cytological and similar samples instead of ethyl alcohol in various titres and xylene which, as already said, is highly toxic and potentially carcinogenic. The components of the composition according to the invention are not highly toxic and there are no indications of presumed carcinogenicity. In practice, for example in the case of preparation of an autoptic and/or histological sample, the composition according to the invention can be used instead of the sequence of ethyl alcohol in various titres and also in place of the xylene in all phases of preparation of the sample and it is possible to perform the dehydration and clarification phase by using the composition only. Furthermore, as already said, use of the composition according to the present invention does not require the use of additional equipment or particular temperature and/or pressure conditions. The dehydration and clarification phases are performed at ambient pressure and temperature without any additional treatment being necessary and therefore they can be easily performed in any working environment and by operators who are not particularly expert. Furthermore, since the application of particular temperatures and pressures is not necessary, the risk of error is reduced and the result is more reliable.

Again according to the present invention it should be highlighted that the isopropyl alcohol is an essential component of the composition subject of the invention.

In particular for the processing phase, from the following tables referring to the processing of so-called "large" samples, it is possible to highlight the advantages obtained from use of the composition according to the present invention.

In the tables given below, a composition indicated as Composition A was used.

Composition A might be obtained mixing the above indicated components. When it will be referred to Composition A in the following examples, it will be intended any possible composition obtained according to the invention, mixing the components as indicated above.

With reference to the enclosed tables, Table 1 shows the procedure commonly adopted for processing of a sample according to the known technique, while Table 2 shows the same procedure using composition A and Table 2A shows a variation of the procedure, again according to the present invention.

**TABLE 1**

| Standard processing protocol | | | |
|---|---|---|---|
| Reagent | Time | Temperature | Vacuum/pressure |
| Alcohol 70° | 1h | Ta | Atmospheric pressure |
| Alcohol 95° | 1h | Ta | Atmospheric pressure |
| Alcohol 95° | 1h | Ta | Atmospheric pressure |
| Alcohol 95° | 1h | Ta | Atmospheric pressure |
| Alcohol 100° | 1h | Ta | Atmospheric pressure |
| Alcohol 100° | 1h | Ta | Atmospheric pressure |
| Alcohol 100° | 1h | Ta | Atmospheric pressure |
| Xylene | 1h 30 min | Ta | Atmospheric pressure |
| Xylene | 1h 30 min | Ta | Atmospheric pressure |
| Paraffin 52-54° | 1h | 55° | Atmospheric pressure |
| Paraffin 52-54° | 1h | 55° | Vacuum/pressure |
| Paraffin 52-54° | 1n | 55° | Vacuum/pressure |
| | 13 h | | |

**TABLE 2**

| Processing protocol with Composition A First case | | | |
|---|---|---|---|
| Reagent | Time | Temperature | Vacuum/pressure |
| Composition A | 2h 30 min | Ta | Atmospheric pressure |
| Composition A | 2h 30min | Ta | Atmospheric pressure |
| Composition A | 2h 30 min | Ta | Atmospheric pressure |
| Composition A | 2h 30 min | Ta | Atmospheric pressure |
| Paraffin 52-54° | 1h | 55° | Atmospheric pressure |
| Paraffin 52-54° | 1h | 55° | Vacuum/pressure |
| Paraffin 52-54° | 1n | 55° | Vacuum/pressure |
| | 13h | | |

**TABLE 2A**

| Processing protocol with Composition A | | | |
|---|---|---|---|
| Second case | | | |
| Reagent | Time | Temperature | Vacuum/pressure |
| Intermediate reagent | 2 min | Ta | Atmospheric pressure |
| Composition A | 2h 30 min | Ta | Atmospheric pressure |
| Composition A | 2h 30min | Ta | Atmospheric pressure |
| Composition A | 2h 30 min | Ta | Atmospheric pressure |
| Composition A | 2h 30 min | Ta | Atmospheric pressure |
| Paraffin 52-54° | 1h | 55° | Atmospheric pressure |
| Paraffin 52-54° | 1h | 55° | Vacuum/pressure |
| Paraffin 52-54° | 1n | 55° | Vacuum/pressure |
| | Approx. 13h | | |

As can be seen, although the overall time necessary for preparation of the samples has not changed, the stages in the various alcohol solutions at different concentrations and the subsequent stages in xylene have been replaced by stages in composition A only. Alternatively, the processing protocol according to the present invention provides for a very quick initial stage involving washing of the sample, prior to dehydration and clarification, in an intermediate reagent which collects the excess formalin left over from the sample fixation phase, traces of which may be still present in the sample. According to the invention, said intermediate reagent is 95% ethyl alcohol.

The composition according to this invention therefore permits dehydration and clarification with one single component, at ambient temperatures and pressure. In this way the processing phase can be performed in fewer stages using a non-toxic non-carcinogenic substance, as opposed to xylene which is necessary in the procedure according to the known technique. Furthermore it is not necessary to perform the dehydration and clarification operations at high temperatures and/or pressures or, at least, at temperatures and/or pressures different from ambient values and it is therefore not necessary to use additional equipment.

Again with reference to the procedure for the preparation of histological and autoptic samples for examination, as already said, the sample must be included in a support that allows it to be cut into sections suitable for examination. The inclusion medium most commonly used is paraffin, both because it is inexpensive and because it can be easily manipulated and very fine sections of tissue can be obtained.

The subsequent stages consisting of inclusion of the sample in paraffin, cutting into the required section, elimination of the supporting paraffin and procedure for staining the sample to be analysed are described below. Also for these subsequent phases, use of the composition according to the invention is fundamental in replacement of the stages involving treatment of the sample with alcohol in various titres and with xylene.

### - Inclusion

Once the samples to be examined have been processed, they are included in blocks of paraffin with melting point 56-58°C by means of steel moulds.

### - Cutting

The sections are cut by using microtomes to obtain sections 3-5 µm thick. These slices of tissue are collected with brushes and laid in a bath containing water at 35°C to allow them to expand.

Subsequently, the sections are collected on slides.

The latter are left to dry in a stove for varying times and at varying temperatures depending on the staining protocol applied subsequently.

### - Paraffin elimination phase

For a section of tissue to be stained, the layer of paraffin that includes it must be eliminated to allow the different reagents to act directly on the tissue.

For this reason the protocol below is adopted; Table 3 shows the procedure commonly followed according to the known technique, Table 4 shows the procedure using Composition A according to the invention and Table 4a shows a variation of the procedure according to the invention.

**TABLE 3**

| Reagent | Time |
|---|---|
| xylene | 10 min |
| Xylene | 10 min |
| Absolute alcohol | 5 min |
| Absolute alcohol | 5 min |
| Alcohol 95° | 2 min |
| Alcohol 95° | 2min |
| Water | 5 min |
| | 39 min |

By replacing the alcohol and xylene treatment, the composition according to the invention permits elimination of the paraffin in two stages only:

**TABLE 4**

| Reagent | Time |
|---|---|
| Composition A | 10 min |
| Composition A | 10 min |
| Water | 3min |
| Water | 3 min |
| | 26 minutes |

**TABLE 4A**

| Reagent | Time |
|---|---|
| Composition A | 10 min |
| Composition A | 10 min |
| Intermediate reagent | 2 min |
| Water | 3min |
| water | 3 min |
| | Approx. 26 minutes |

In this variation, the paraffin is eliminated in three stages only, one of which
- the intermediate reagent stage - is quick and simple.
- Diaphanisation

After performing any staining protocol, the section must be dehydrated again for application of the cover by means of a resinous mounting medium. This ensures longer life of the preserved section.

To perform this further stage the protocol below is usually applied, which shows the procedure according to the known art, compared with the phases shown in Table 6 in which Composition A according to the present invention is used.

**TABLE 5**

| Reagent | Time |
|---|---|
| Alcohol 70° | 10 sec |
| Alcohol 80° | 10sec |
| Alcohol 95° | 1 min |
| Alcohol 95° | 1 min |
| Absolute alcohol | 2 min |
| Absolute alcohol | 2min |
| Xylene | 5min |
| xylene | 5min |
| | 16 min 20 sec |

By replacing the alcohols and xylene, the composition according to the present invention also permits performance of the diaphanisation phase in two stages only:

**TABLE 6**

| Reagent | Time |
|---|---|
| Composition A | 5 min |
| Composition A | 5 min |
| | 10 min |

In addition to the above, the composition according to the invention can also be used in any histological staining protocol requiring intermediate stages in alcohols at different percentages alongside any treatment with xylene, at times maintaining the same stage duration but guaranteeing a clear improvement as regards the manual aspect of the method and ease of execution.

Tables 7, 8 and 8A show the Hematoxylin/Eosin staining methods according to the known technique and using the composition according to the present invention, in two different variations.

**TABLE 7**

| PROTOCOL ACCORDING TO THE TRADITIONAL SYSTEMS | |
|---|---|
| STAINING PROTOCOL | TIME IN |
| IMMERSE THE SECTIONS IN XYLENE | 10 |
| IMMERSE THE SECTIONS IN XYLENE | 10 |
| IMMERSE THE SECTIONS IN ABSOLUTE ALCOHOL | 2 |
| IMMERSE THE SECTIONS IN ABSOLUTE ALCOHOL | 2 |
| IMMERSE THE SECTIONS IN 95° ALCOHOL | 2 |
| IMMERSE THE SECTIONS IN 80° ALCOHOL | 2 |
| STAGE IN DISTILLED WATER | 5 |
| IMMERSE THE SECTIONS IN HEMATOXYLIN | 5 |
| COLOUR CHANGE UNDER RUNNING WATER | 5 |
| IMMERSE THE SECTIONS IN ALCOHOLIC EOSIN | 1 |
| IMMERSE THE SECTIONS IN 70° ALCOHOL | 1 |
| IMMERSE THE SECTIONS IN 80° ALCOHOL | 1 |
| IMMERSE THE SECTIONS IN 95° ALCOHOL | 1 |
| IMMERSE THE SECTIONS IN ABSOLUTE ALCOHOL | 2 |
| IMMERSE THE SECTIONS IN ABSOLUTE ALCOHOL | 2 |
| IMMERSE THE SECTIONS IN XYLENE | 5 |
| IMMERSE THE SECTIONS IN XYLENE | 5 |
| MOUNT THE SLIDES WITH ANY MOUNTING MEDIUM | |
| | 61 MIN. |

**TABLE 8**

| PROTOCOL USING COMPOSITION A | |
|---|---|
| STAINING PROTOCOL | TIME(MIN) |
| IMMERSE THE SECTIONS IN Composition A | 10 |
| IMMERSE THE SECTIONS IN Composition A | 10 |
| STAGE IN DISTILLED WATER | 5 |
| IMMERSE THE SECTIONS IN HEMATOXYLIN | 5 |
| COLOUR CHANGE UNDER RUNNING WATER | 5 |
| IMMERSE THE SECTIONS IN ALCOHOLIC EOSIN | 1 |
| IMMERSE THE SECTIONS IN Composition A | 5 |
| MOUNT THE SLIDES WITH ANY MOUNTING MEDIUM | |
| | 41 MIN. |

**TABLE 8A**

| PROTOCOL USING COMPOSITION A | |
|---|---|
| STAINING PROTOCOL | TIME |
| IMMERSE THE SECTIONS IN Composition A | 10 |
| IMMERSE THE SECTIONS IN Composition A | 10 |
| INTERMEDIATE REAGENT | 2 |
| STAGE IN DISTILLED WATER | 5 |
| IMMERSE THE SECTIONS IN HEMATOXYLIN | 5 |
| COLOUR CHANGE UNDER RUNNING WATER | 5 |
| IMMERSE THE SECTIONS IN ALCOHOLIC EOSIN | 1 |
| INTERMEDIATE REAGENT | 30 sec. |
| IMMERSE THE SECTIONS IN Composition A | 5 |
| MOUNT THE SLIDES WITH ANY MOUNTING MEDIUM | |
| | Approx. 43 MIN. |

In this case, in addition to the advantages already described, also a considerable saving in time when the procedure is performed using the composition according to the invention. Moreover, according to the variation shown in Table 8A, the brief washing in intermediate reagent avoids any excess staining.

### - Cytological samples

The composition according to the invention can be advantageously employed also in the preparation of cytological samples, fixed in alcohol and ether for example and not included in paraffin or other inclusion agents, such as urine, smear tests, sputum and other liquid or other types of samples.

The composition according to the invention replaces:
1. All the washing processes in alcohol at different concentrations, while maintaining the same overall washing time or reducing it if necessary, as required.
2. The final diaphanisation process.

Tables 9, 10 and 10A show examples of PAPANICOLAOU staining according to the known technique and using the composition according to the present invention, in two possible variations.

**TABLE 9**

| TRADITIONAL PROTOCOL ACCORDING TO THE KNOWN ART | |
|---|---|
| STAINING PROTOCOL | TIME (MIN) |
| AFTER FIXING THE SMEAR WITH APPROPRIATE FIXATIVES IMMERSE THE SECTIONS IN 95° ALCOHOL | 1 |
| IMMERSE THE SECTIONS IN 80° ALCOHOL | 1 |
| IMMERSE THE SECTIONS IN 70° ALCOHOL | 1 |
| IMMERSE THE SECTIONS IN 50° ALCOHOL | 1 |
| IMMERSE THE SECTIONS IN FRESH WATER | 5 |
| IMMERSE THE SLIDES IN DISTILLED WATER | 1 |
| IMMERSE THE SECTIONS IN HEMATOXYLIN | 5 |
| COLOUR CHANGE UNDER RUNNING WATER | 5 |
| IMMERSE THE SECTIONS IN 70° ALCOHOL | 1 |
| IMMERSE THE SECTIONS IN 95° ALCOHOL | 1 |
| IMMERSE THE SECTIONS IN 95° ALCOHOL | 1 |
| IMMERSE THE SECTIONS IN ORANGE G | 3 |
| IMMERSE THE SECTIONS IN 95° ALCOHOL | 30 SECONDS |
| IMMERSE THE SECTIONS IN 95° ALCOHOL | 30 SECONDS |
| IMMERSE THE SECTIONS IN EA50 | 5 |
| IMMERSE THE SECTIONS IN 95° ALCOHOL | 30 SECONDS |
| IMMERSE THE SECTIONS IN 95° ALCOHOL | 30 SECONDS |
| IMMERSE THE SECTIONS IN ABSOLUTE ALCOHOL | 2 |
| IMMERSE THE SECTIONS IN ABSOLUTE ALCOHOL | 2 |
| IMMERSE THE SECTIONS IN XYLENE | 5 |
| IMMERSE THE SECTIONS IN XYLENE | 5 |
| MOUNT THE SLIDES WITH ANY MOUNTING MEDIUM | |
| | 47MINUTES |

**TABLE 10**

| PROTOCOL USING COMPOSITION A | |
|---|---|
| STAINING PROTOCOL | TIME (MIN) |
| AFTER FIXING THE SMEAR WITH APPROPRIATE FIXATIVES, IMMERSE THE SAMPLE IN Composition A | 5 |
| IMMERSE THE SAMPLE IN FRESH WATER | 5 |
| IMMERSE THE SAMPLE IN DISTILLED WATER | 1 |
| IMMERSE THE SAMPLE IN HEMATOXYLIN | 5 |
| COLOUR CHANGE UNDER RUNNING WATER | 5 |
| IMMERSE THE SECTIONS IN Composition A | 3 |
| IMMERSE THE SECTIONS IN ORANGE G | 3 |
| IMMERSE THE SECTIONS IN Composition A | 1 |
| IMMERSE THE SECTIONS IN EA50 | 5 |
| IMMERSE THE SECTIONS IN Composition A | 5 |
| MOUNT THE SLIDES WITH ANY MOUNTING MEDIUM | |
| | 38 MINUTES |

**TABLE 10A**

| PROTOCOL USING COMPOSITION A | |
|---|---|
| STAINING PROTOCOL | TIME (MIN) |
| AFTER FIXING THE SMEAR WITH APPROPRIATE FIXATIVES, IMMERSE THE SAMPLE IN Composition A | 5 |
| IMMERSE THE SAMPLE IN FRESH WATER | 5 |
| IMMERSE THE SAMPLE IN DISTILLED WATER | 1 |
| IMMERSE THE SAMPLE IN HEMATOXYLIN | 5 |
| COLOUR CHANGE UNDER RUNNING WATER | 5 |
| IMMERSE THE SECTIONS IN Composition A | 3 |
| IMMERSE THE SECTIONS IN ORANGE G | 3 |
| IMMERSE THE SECTIONS IN Composition A | 1 |
| IMMERSE THE SECTIONS IN EA50 | 5 |
| INTERMEDIATE REAGENT | 30 sec |
| IMMERSE THE SECTIONS IN Composition A | 5 |
| MOUNT THE SLIDES WITH ANY MOUNTING MEDIUM | |
| | Approx. 38 MINUTES |

Also in this case, any excess staining can be eliminated by quick washing in the intermediate reagent.

In this case, even if there is no significant saving in time, the saving in terms of fewer stages performed and fewer reagents used is obvious.

In all the above cases exemplified in the tables, the term "intermediate reagent" refers to 95% ethyl alcohol.

As already said above, the composition according to the present invention allows for the preparation of autoptic, histological and cytological samples without the xylene and alcohol stages, thus avoiding the repeated use of different reagents at different times and according to fixed sequences. It is obvious that when the operator is obliged to use many different reagents according to a pre-set order, he can easily become confused and can commit errors that affect the final result of the analysis.

The composition according to the present invention also permits reduction of the paraffin elimination times in the corresponding stage, thanks to the considerable affinity between the composition itself, the paraffin and the other reagents used.

The same composition does not alter the morphological characteristics and the antigenic expression of the tissue to which it confers brilliance.

As already said, use of the composition according to the present invention allows the phases described to be performed at ambient temperature and pressure and therefore does not require particular equipment or additional processes.

If the preparation is performed on a suitable sample, the composition used and deriving from the various phases of the procedure can be recovered and, after adequate purification, re-used if necessary. Naturally any recycling after purification must be compatible with the type of potential pollution of the composition, which depends mainly on the pathologies encountered in the subjects from which the sample is taken, said sample having been prepared and having come into contact with the composition according to the invention.

## Claims

1. Process for processing a histological, autoptic or cytological sample **characterized by** treating said sample, at room temperature and room pressure, with a composition comprising octane 60% by volume, isopropyl alcohol 10% by volume and ethyl alcohol 30% by volume.

2. Process according to claim 1, **characterized in that** a washing of the sample with an intermediate reagent is carried out before processing said sample with said composition.

3. Process according to claim 2, wherein said intermediate reagent is 95% ethyl alcohol.

4. Composition comprising octane 60% by volume, isopropyl alcohol 10% by volume and ethyl alcohol 30% by volume.

5. Composition according to claim 4, for its use in processing a histological, autoptic or cytological sample.

6. Use of a composition according to claim 4 for processing a histological, autoptic or cytological sample.

## Patentansprüche

1. Ein Verfahren zur Behandlung einer histologischen oder cytologischen Probe oder einer Autopsieprobe, **gekennzeichnet durch** die Behandlung der Probe bei Umgebungstemperatur und Umgebungsdruck mit einer Zusammensetzung enthaltend 60 Vol.-% Oktan, 10 Vol.-% Isopropylalkohol und 30 Vol.-% Ethylalkohol.

2. Das Verfahren nach Anspruch 1, **dadurch gekennzeichnet, dass** vor der Behandlung der Probe mit der Zusammensetzung die Probe mit einem intermediären Reagens gewaschen wird.

3. Das Verfahren nach Anspruch 2, wobei das intermediäre Reagens eine 95 %ige Ethylalkohollösung ist.

4. Eine Zusammensetzung enthaltend 60 Vol.-% Oktan, 10 Vol.-% Isopropylalkohol und 30 Vol.-% Ethylalkohol.

5. Die Zusammensetzung gemäß Anspruch 4 zur Verwendung bei der Behandlung von histologischen oder cytologischen Proben oder von Autopsieproben.

6. Verwendung der Zusammensetzung nach Anspruch 4 zur Behandlung einer histologischen oder cytologischen Probe oder einer Autopsieprobe.

## Revendications

1. Procédé de traitement d'un échantillon histologique, autoptique ou cytologique, **caractérisé en ce qu'**il consiste à traiter ledit échantillon, à température ambiante et à pression ambiante, avec une composition comprenant 60 % d'octane en volume, 10 % d'alcool isopropylique en volume et 30 % d'alcool éthylique en volume.

2. Procédé selon la revendication 1, **caractérisé en ce qu'**on effectue un lavage de l'échantillon avec un réactif intermédiaire avant de traiter ledit échantillon avec ladite composition.

3. Procédé selon la revendication 2, dans lequel ledit réactif intermédiaire est de l'alcool éthylique à 95 %.

4. Composition comprenant 60 % d'octane en volume, 10 % d'alcool isopropylique en volume et 30 % d'alcool éthylique en volume.

5. Composition selon la revendication 4, utilisable pour traiter un échantillon histologique, autoptique ou cytologique.

6. Utilisation d'une composition selon la revendication 4 pour traiter un échantillon histologique, autoptique ou cytologique.
